# EUROPEAN PATENT SPECIFICATION

(11) **EP 3 639 770 B1**
(45) Date of publication and mention of the grant of the patent: **28.07.2021**
(21) Application number: 19203738.0
(22) Date of filing: 17.10.2019
(51) Int. Cl.: A61B 17/221, A61M 25/00, A61M 25/01

(54) **CATHETER**
KATHETER
CATHÉTER

(30) Priority: 19.10.2018 JP 2018197336
(43) Date of publication of application: 22.04.2020
(73) Proprietor: ASAHI INTECC CO., LTD., Seto-shi, Aichi 489-0071 (JP)
(72) Inventor: NAKAGAWA, Yuta, Seto-shi, Aichi 489-0071 (JP); KAWAGUCHI, Keisuke, Seto-shi, Aichi 489-0071 (JP)
(74) Representative: Winter, Brandl - Partnerschaft mbB

(56) References cited:
- US-A1- 2006 058 813
- US-A1- 2011 319 917
- US-A1- 2017 105 742
- US-A1- 2017 224 380

## Description

The present disclosure relates to a catheter.

There are medical devices for removing a blood vessel-occluding blockage such as choronic total occlusion (CTO) to improve a blood flow. A medical device according to JP3655920 B2 include, for example, those in which meshed-like braided wires will be radially expanded at a site where a blockage is present within a blood vessel to remove the blockage.

In the medical device according to JP3655920 B2 where a guide wire is fit into a bearing surface, pulling the guide wire will cause the bearing surface to press against a ring body provided at the front end of wires, leading to radial expansion of the wire.

However, the front end of the guide wire in the medical device according to JP3655920 B2 is merely fit into the bearing surface, but not fixed firmly to the bearing surface. Further, the bearing surface, which is composed of a bead, solder, or the like, can not ensure the flexibility of the front end portion of the medical device.

US2017/105742 A1 discloses a catheter comprising a hollow shaft, a tubular mesh member joined to a distal end of the hollow shaft, a distal tip joined to a distal end of the mesh member, and a core wire joined to the distal tip. The tubular mesh member is configured to expand and contract in a radial direction. The core wire is configured to control an expansion and contraction of the tubular mesh member.

An object of the present invention is to provide a catheter in which a front end tip can be fixed firmly to a core wire, and the flexibility of a front end portion can be ensured.

To achieve the above object, a catheter according to claim 1 is defined. Preferred embodiments are defined in the dependent claims.

An embodiment of the present disclosure has a long axis and includes: a hollow shaft having a front end and a base end; a mesh member with a tubular shape, the mesh member having a base end joined to the front end of the hollow shaft, and being configured to expand and contract in a radial direction perpendicular to the long axis of the catheter; a front end tip joined to a front end of the mesh member; and a core wire having a long axis and a front end portion joined to the front end tip.

The core wire extends through an inside of the mesh member and the hollow shaft, a base end of the core wire is located on a base end side relative to a base end of the hollow shaft, which means that the core wire penetrates through the mesh member and the hollow shaft.

In the embodiment of the present disclosure, the front end portion of the core wire is formed with, i.e., comprises one or more holes, and a portion of the front end tip enters into at least a portion of the one or more holes.

The one or more holes may be one or more through-holes, and a portion of the front end tip may enter into at least a portion of the one or more through-holes. The portion of the front end tip may penetrate through the one or more through-holes.

One through-hole may be formed to become wider toward a front end of the one through-hole. In other words, the width of the one through-hole may increase toward a front end of the core wire.

The one or more holes may be one or more depressed portions, and a portion of the front end tip may enter into at least a portion of the one or more depressed portions.

Each of the one or more depressed portions may be depressed (extend) in a direction intersecting the long axis of the core wire.

The front end of the front end portion may be covered by the front end tip. The front end portion may have a plate shape.

The front end portion of the core wire may be tapered to become thinner toward a front end of the front end portion.
Fig. 1 is a schematic cross-sectional view of a catheter according to a first embodiment, showing a state where a mesh member is contracted.
Fig. 2A shows a schematic planar view of a core wire, and Fig. 2B shows a schematic side view of the core wire.
Fig. 3 is an enlarged cross-sectional view of the front end portion of the catheter according to the first embodiment.
Fig. 4 is a schematic cross-sectional view of the catheter according to a first embodiment, showing a state where a mesh member is expanded.
Fig. 5 shows a variation of a through-hole of the core wire.
Fig. 6 shows a variation of a through-hole of the core wire.
Fig. 7 shows a variation of through-holes of the core wire.
Fig. 8 shows a variation of through-holes of the core wire.
Fig. 9 shows an enlarged cross-sectional view of a front end portion of a catheter according to a second embodiment.
Fig. 10A shows a schematic planar view of a core wire, and Fig. 10B shows a schematic cross-sectional view of the core wire along the Xb-Xb line shown in Fig. 2A.
Fig. 11 shows a variation of depressed portions of the core wire.
Fig. 12 shows a variation of the front end portion of the core wire.

The catheters according to the embodiments of the present disclosure will be described with reference to the drawings.

It is noted that among guide wires, the term "antegrade guide wire" as used herein means a guide wire to be pushed through toward an operation area such as an occlusion site in a blood vessel prior to a catheter while the term "retrograde guide wire" means a guide wire approaching toward a catheter from a front end side of the catheter through, for example, a blood vessel.

Further, the term "frond end side" as used herein refers to a direction where a front end tip is located relative to a mesh member along a longitudinal direction of a catheter. The term "base end side" refers to a direction which is opposite to the front end side along the above longitudinal direction. The term "front end" refers to an end portion in the front end side of each member of a catheter. The term "base end" refers to an end portion in the base end side of each member of a catheter.

### [First embodiment]

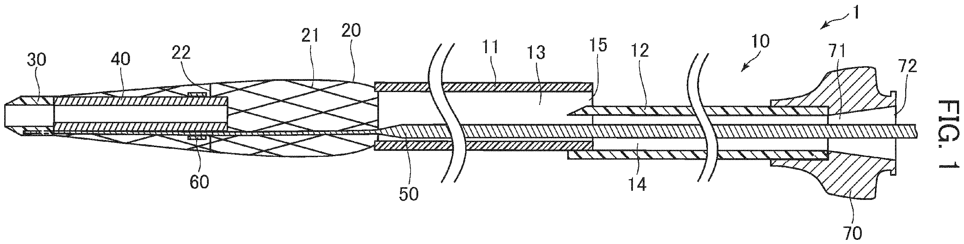

Fig. 1 is a schematic cross-sectional view of a catheter 1 according to a first embodiment, showing a state where a mesh member 20 is contracted.

In Fig. 1, the left side in the figure corresponds to the front end side (the distal side) which is to be inserted into the body, and the right side corresponds to the base end side (the hand side, the proximal side) which is to be operated by an operator such as a physician.

As shown in Fig. 1, the catheter 1 includes a first hollow shaft 10, a mesh member 20, a front end tip 30, a second hollow shaft 40, a core wire 50, a marker 60, and a connector 70.

The first hollow shaft 10 has a front end side shaft 11 and a base end side shaft 12. The front end of the front end side shaft 11 is connected to the base end of the mesh member 20. The front end of the base end side shaft 12 is connected to the base end of the front end side shaft 11. The connector 70 is connected to the base end of the base end side shaft 12.

The front end side shaft 11 has a lumen 13 through which an antegrade guide wire, a retrograde guide wire, and a core wire 50 can be inserted and passed. The base end side shaft 12 has a lumen 14 through which the core wire 50 can be inserted and passed. In the connection between the front end side shaft 11 and the base end side shaft 12, the front end side shaft 11 and the base end side shaft 12 together form a guide wire port 15 opened toward the base end side. A retrograde guide wire will be sent out to the outside of the catheter 1 through the guide wire port 15.

A material for the first hollow shaft 10 preferably has antithrombogenicity, flexibility, and biocompatibility because the first hollow shaft 10 is to be inserted into and passed through a blood vessel. Examples include resin materials or metal materials. For the front end side shaft 11, a resin material is preferred because flexibility is required. For example, polyamide resin, polyolefin resin, polyester resin, polyurethane resin, silicone resin, fluororesin, and the like may be used. For the base end side shaft 12, a metal material is preferred because pushability is required. For example, stainless steel such as SUS304, nickel-titanium alloy, cobalt-chromium alloy, and the like may be used.

The mesh member 20 is a tubular member capable of expanding and contracting in the radial direction thereof. When the core wire 50 described below is pulled toward the base end side, the mesh member 20 undergoes out-of-plane deformation and inflates outwardly in the radial direction to expand radially. The catheter 1 receives a retrograde guide wire through a mesh opening formed in this radially expanded mesh member 20.

In the present embodiment, the mesh member 20 is configured so that a plurality of wires 21 are braided in a lattice-like pattern to form a tubular shape. Further, the mesh member 20 has the mesh opening between adjacent braided wires, and receives a retrograde guide wire through the mesh opening which is enlarged when the mesh member 20 radially expands. It is noted that the front end and the base end of each of the wires 21 of the mesh member 20 are joined to the front end tip 30 and the first hollow shaft 10, respectively.

Here, each of the wires 21 of the mesh member 20 may be formed of either a single wire or multiple wires, or may be formed of a twisted wire where a plurality of metal wires are twisted, the metal wires being different, for example, in diameter and others.

As a material for each of the wires 21 of the mesh member 20, a metal material or a resin material may be used. Resin materials include polyamide, polyester, polyarylate, polyetheretherketone, and the like. It is noted that metal materials are preferred in view of improving strength and flexibility. Such metal materials include, for example, stainless steel such as SUS304, nickel-titanium alloy, cobalt-chromium alloy, and the like. It is noted that each of the wires 21 may be formed of the same material or different materials.

Further, a material for each of the wires 21 of the mesh member 20 may be a radiopaque material in view of improving the visibility of the mesh member 20. Such radiopaque materials include, for example, gold, platinum, tungsten, or alloys including these elements (for example, platinum-nickel alloy and the like). It is noted that a radiopaque material may be combined with a material other than the radiopaque material, such as a composite where a radiopaque material is coated on a non-radiopaque material.

A guiding film 22 is provided on the mesh member 20. The front end of the guide film 22 is located between the base end of the front end tip 30 and the front end of the first hollow shaft 10. The guiding film 22 serves to smoothly direct a retrograde guide wire received through the mesh opening of the mesh member 20 toward the first hollow shaft 10. The guiding film 22 has a front end located at a substantially mid-portion of the mesh member 20 in a long axis direction of the catheter 1, and a base end located in the front end of the first hollow shaft 10. The guiding film 22 is formed over the mesh member 20 so as to crosslink adjacent wires 21. Here, the guiding film 22 unfolds into a funnel shape when the mesh member 20 radially expands. This enables the retrograde guide wire to be directed into the first hollow shaft 10 through the mesh member 20. It is sufficient that at least a portion of the guiding film 22 (for example, the front end outer periphery of the guiding film 22, and the like) is joined to the mesh member 20. For example, the guiding film 22 may be a film-like material (not shown).

Materials which can be used for the guiding film 22 include, for example, polyethylene, polyurethane, polyamide, polyamide elastomer, polyolefin, polyester, polyester elastomer, and the like. Among these, the above material is preferably polyurethane in view of improving surface slidability. Further, there is no particular limitation for a method of forming the guiding film 22, but the followings may be used: for example, a dip method in a case where a guiding film is to be arranged on the mesh member 20; a method involving fusing the front end of a film with the mesh member 20 in a case of a film-like guide coating; and others.

The front end tip 30 is connected to the front end of the mesh member 20. Specifically, the front end tip 30 is formed so as to be sharpened toward the front end side, and the front end portion of each of the wire 21 of the mesh member 20, the front end portion of the second hollow shaft 40, and the front end portion of the core wire 50 are embedded into the base end of the the front end tip 30.

A material for the front end tip 30 preferably has softness because the catheter 1 is intended to advance through the inside of a blood vessel. Such materials having softness include, for example, resin materials such as polyurethane and polyurethane elastomer; and the like.

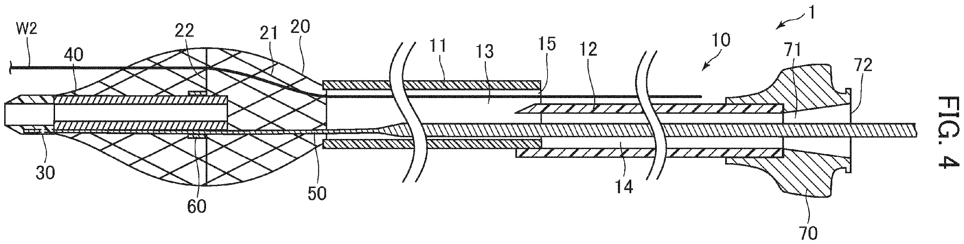

The second hollow shaft 40 is connected to the front end tip 30, and protruded to the base end side in a space inside the mesh member 20. As shown in Fig. 1, the base end of the second hollow shaft 40 is located between the front end of the first hollow shaft 10 and the base end of the front end tip 30 in the space inside the mesh member 20. The second hollow shaft 40 is configured so that the base end thereof is positioned between the front end of the first hollow shaft 10 and the base end of the front end tip 30 in the space inside the mesh member 20 when the mesh member 20 is expanded radially and outwardly in the radial direction as shown in Fig. 4. This enables a retrograde guide wire to be easily received into the first hollow shaft 10.

The second hollow shaft 40 is also intended to be inserted into and passed through a blood vessel as is the aforementioned first hollow shaft 10. Therefore, a material for the second hollow shaft 40 preferably has antithrombogenicity, flexibility, and biocompatibility. Such materials include, for example, those exemplified in the description of the first hollow shaft 10, but resin materials are preferred in view of flexibility.

The core wire 50 has a front end connected to the front end tip 30 and a based end extending to the outside through the connector 70 located in the base end side of the first hollow shaft 10. This means that the core wire 50 penetrates through the mesh member 20, the lumens 13 and 14 of the first hollow shaft 10, and a communication hole 71 of the connector 70. It is noted that when an operator operates the core wire 50 from the outside of the connector 70, the core wire 50 advances and retracts along the long axis direction, enabling the mesh member 20 to expand and contract in the radial direction.

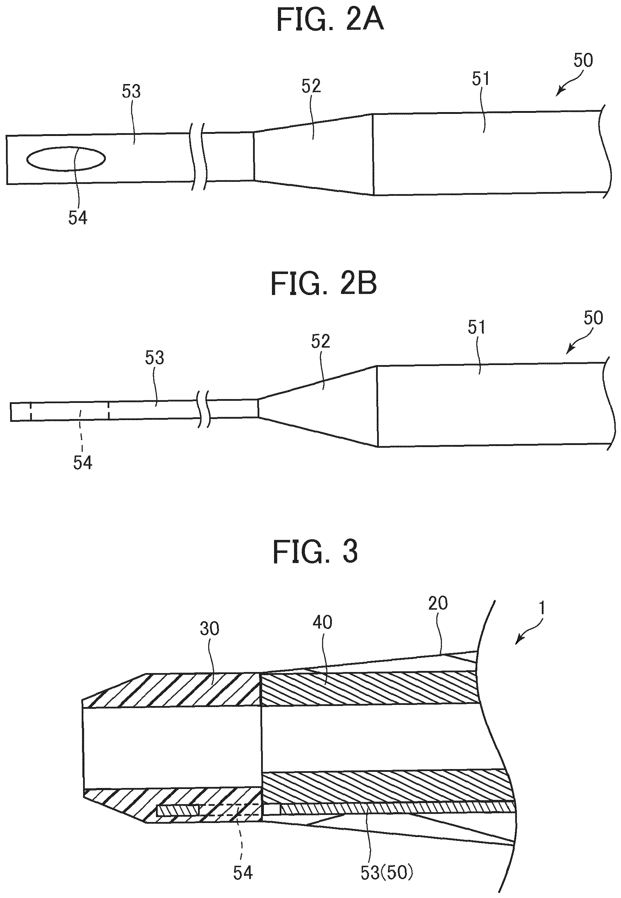

Fig. 2A shows a schematic planar view of the core wire 50, and Fig. 2B shows a schematic side view of the core wire 50.

Fig. 3 shows an enlarged cross-sectional view of the front end portion of the catheter 1.

The core wire 50 has a base end portion 51, a tapered portion 52, and a front end portion 53.

The base end portion 51 is located in the base end side of the catheters 1. The base end of the base end portion 51 is located outside of the connector 70. Further, the base end portion 51 has a substantially constant outer diameter from the base end through the front end thereof.

The tapered portion 52 is located on the front end side of the base end portion 51, and extends from the front end of the base end portion 51 to the front end side, and is configured so that the outer shape is tapered toward the front end side.

The front end portion 53 is located on the front end side of the tapered portion 52, and extends from the front end of the tapered portion 52 to the front end side. The front end portion 53 has a plate-like shape having substantially constant thickness from the base end through the front end. A through-hole 54 having an elliptical shape is formed near the front end of the front end portion 53.

As shown in Fig. 3, a portion of the front end tip 30 enters into the through-hole 54 of the front end portion 53. The base end of the through-hole 54 is located at the base end side relative to the base end of the front end tip 30 in the present embodiment, but the base end of the through-hole 54 may be located at the front end side relative to the base end of the front end tip 30. That is, a portion of the front end tip 30 may enter into at least a portion of the through-hole 54. A portion of the front end tip 30 may also penetrate through the through-hole 54.

A material for the core wire 50 preferably has sufficient tensile strength and stiffness in view of preventing breakage of the core wire 50 itself and ensuring reliable expansion and contraction of the mesh member 20. Such metal materials include, for example, metal materials such as stainless steel such as SUS304, nickel-titanium alloy, cobalt-chromium alloy; and the like.

The marker 60 has a substantially annular or substantially C-like shape as viewed in a cross-section, and covers the second hollow shaft 40 and the core wire 50. The marker 60 can prevent separation of the base end of the second hollow shaft 40 from the core wire 50, enabling them to be moved together.

The marker 60 may be formed of resin materials such as polyamide resin, polyolefin resin, polyester resin, polyurethane resin, silicone resin, and fluororesin; or metal materials such as stainless steel such as SUS304, nickel-titanium alloy, and cobalt-chromium alloy. When formed of a resin, the resin preferably contains a radiopaque material such as bismuth trioxide, tungsten, and barium sulfate. When formed of a metal material, a radiopaque material such as platinum and tungsten is preferably used. The marker 60 is preferably located near the front end of the guiding film 22 upon extension of the mesh member 20.

The connector 70 serves as a member with which an operator holds the catheter 1. As shown in Fig. 1, the connector 70 is connected to the base end of the first hollow shaft 10, and has the communication hole 71 in communication with the lumens 13, 14 of the first hollow shaft 1, and an opening 72 formed in the base end of the communication hole 71 so that the core wire 50 can be exposed to the outside. It is noted that there is no particular limitation for the shape of the connector 70, and any shape may be used as long as an operator can easily hold it.

Next, an example of the operating mode of the aforementioned catheter 1 will be described.

First, an antegrade guide wire W1 (not shown) is inserted into, for example, a blood vessel, and then pushed through to a site where an blockage is present along with a blood vessel (hereinafter may also be referred to as an "occlusion site"). After the front end of the antegrade guide wire W1 reaches the occlusion site, a balloon catheter (not shown) is inserted through to the occlusion site using the antegrade guide wire W1 as a guide. Then the balloon is inflated radially to expand the occlusion site. After the occlusion site is expanded, the balloon is deflated radially, and the balloon catheter is then withdrawn out of the blood vessel.

Next, the antegrade guide wire W1 is inserted into and passed through the catheter 1 so that the base end of the antegrade guide wire W1 exits the guide wire port 15 to the outside of the catheter 1 thorough an opening of the front end tip 30, the through-holes of the front end tip 30 and the second hollow shaft 40, the space inside the mesh member 20, and then the lumen 13 of the front end side shaft 11. The front end of the catheter 1 is then pushed to the occlusion site expanded by the balloon catheter in the blood vessel using the antegrade guide wire W1 as a guide. At that time, the catheter 1 in a state where the mesh member 20 remains contracted radially is inserted into the blood vessel, the above contracted state is maintained until the front end of the catheter 1 reaches the occlusion site.

After the front end of the catheter 1 reaches the occlusion site, the antegrade guide wire W1 is pulled out of the catheter 1 by pulling the antegrade guide wire W1 to the base end side. Subsequently, a gap between the front end of the mesh member 20 and the front end of the first hollow shaft 10 becomes narrow by pulling the core wire 50 exposed outside the connector 70 toward the base end side. As a result of this, the mesh member 20 undergoes out-of-plane deformation outwardly in the radial direction to expand radially. At that time, the guiding film 22 is radially expanded as the mesh member 20 is radially expanded, transforming the guiding film 22 into an overall funnel-like shape because the front end of the guiding film 22 is joined to the substantially mid-portion of the mesh member 20 in the long axis direction in this embodiment. It is noted that the mesh opening is also expanded as the mesh member 20 is radially expanded, leading to a condition where a retrograde guide wire W2 can easily be received. Further, the second hollow shaft 40 is bound at the base end thereof by the core wire 50 through the marker 60, and thus the second hollow shaft 40 moves along the long axis direction (longitudinal direction) of the catheter 1 without causing inclination of the second hollow shaft 40. It is noted that after the front end of the catheter 1 advances along the antegrade guide wire W1, and reaches an occlusion site, the mesh member 20 may be radially expanded, and then the antegrade guide wire W1 may be pulled out of the catheter 1 by pulling the antegrade guide wire W1 to the base end side.

Next, the retrograde guide wire W2 approaching toward the catheter 1 from the front end side of the catheter 1 is received in the catheter 1 as shown in Fig. 4. An approaching route of the aforementioned retrograde guide wire W2 may likely be, for example, via a false lumen within a blood vessel wall surrounding an occlusion site, a through-hole penetrating an occlusion site, or the like, but the retrograde guide wire W2 can approach via any route. The retrograde guide wire W2 is received in the space inside the mesh member 20 through the mesh opening of the mesh member 20 expanded radially, and then inserted into and passed through the front end side shaft 11 of the first hollow shaft 10, and sent out to the outside of the catheter 1 through the guide wire port 15.

As described above, a portion of the front end tip 30 enters into the through-hole 54 formed on the front end portion 53 of the core wire 50 in the catheter 1. This enables the front end tip 30 to be fixed firmly to the core wire 50. Further, the flexibility of the front end portion of the catheter 1 can be ensured by forming a hole on the front end portion 53 of the core wire 50.

Further, a hole formed on the front end portion 53 of the core wire 50 in the present embodiment is the through-hole 54. This enables the front end tip 30 to be fixed more firmly to the core wire 50.

Further, by virtue of the plate-like shape of the front end portion 53, a portion of the front end tip 30 can unfailingly enter into the through-hole 54, and the both faces of the front end portion 53 can easily be covered. This, in turn, enables the front end tip 30 to be fixed more firmly to the core wire 50.

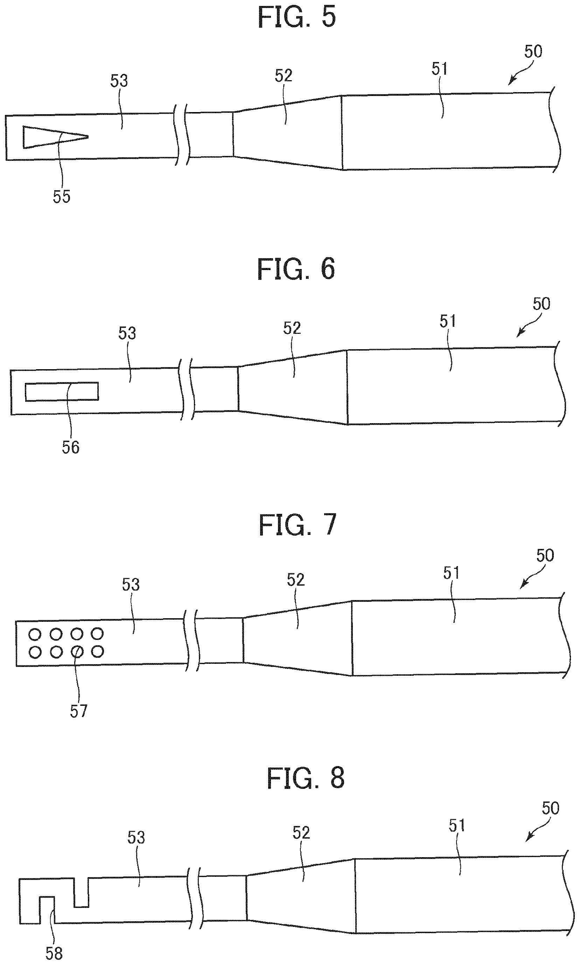

Moreover, the shape of a through-hole formed on the front end portion 53 may be a substantially triangular through-hole 55 formed so as to become wider toward the front end (Fig. 5), or may be a rectangular through-hole 56 (Fig. 6), or may be a plurality of through-holes 57 (Fig. 7), or may be a plurality of notched through-holes 58 (Fig. 8). The substantially triangular through-hole 55 formed so as to become wider toward the front end can improve the flexibility of the front end portion of the catheter 1.

### [Second embodiment]

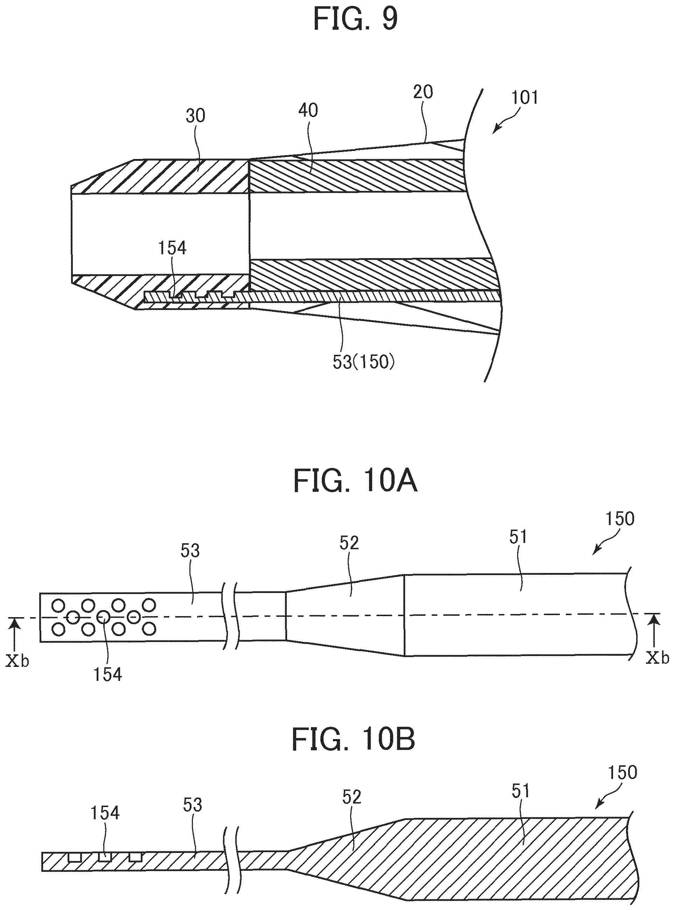

Fig. 9 shows an enlarged cross-sectional view of a front end portion of a catheter 101 according to a second embodiment.

In Fig. 9, the left side in the figure corresponds to the front end side (the distal side) which is to be inserted into the body, and the right side corresponds to the base end side (the hand side, the proximal side) which is to be operated by an operator such as a physician.

The catheter 101 includes the first hollow shaft 10 (Fig. 1), the mesh member 20, the front end tip 30, the second hollow shaft 40, a core wire 150, the marker 60 (Fig. 1), and the connector 70 (Fig. 1). In the catheter 101, the configuration of the core wire 150 is different from that of the core wire 50 of the first embodiment. The configurations of the first hollow shaft 10, the mesh member 20, the front end tip 30, the second hollow shaft 40, the marker 60, and the connector 70 are similar to those of the first embodiment. Therefore, description thereof will be omitted.

Fig. 10A shows a schematic planar view of the core wire 150, and Fig. 10B shows a schematic cross-sectional view of the core wire 150 along the Xb-Xb line shown in Fig. 10A.

The core wire 150 has a configuration different from that of the core wire 50 of the first embodiment in that depressed holes are formed instead of through-holes. The core wires 150 has the same configuration as the core wire 50 of the first embodiment except for the holes. As shown in Fig. 10, a plurality of depressed portions 154 each having a circular shape in a planar view are formed on the front end of the front end portion 53 of the core wire 150. As shown in Fig. 9, a portion of the front end tip 30 enters into each of the plurality of depressed portions 154. It is noted that among the plurality of depressed portions 154, some depressed portions 154 located on the base end side may be located on the base end side relative to the base end of the front end tip 30, or may be located on the front end side relative to the base end of the front end tip 30. That is, a portion of the front end tip 30 may enter into at least a portion of the plurality of depressed portions 154.

As described above, a portion of the front end tip 30 enters into the depressed portions 154 formed on the front end portion 53 of the core wire 150 in the catheter 101. This enables the front end tip 30 to be fixed firmly to the core wire 150.

Further, by virtue of a plate-like shape of the front end portion 53, a portion of the front end tip 30 can unfailingly enter into the depressed portions 154, and the both faces of the front end portion 53 can easily be covered. This, in turn, enables the front end tip 30 to be fixed more firmly to the core wire 150.

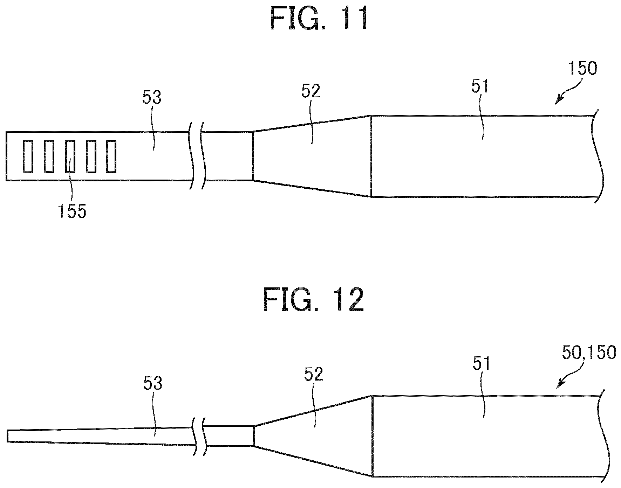

Moreover, the depressed portions formed on the front end portion 53 may be depressed portions 155 (Fig. 11), each of which is configured to extend in a direction intersecting the long axis direction of the core wire 150. The above depressed portions 155 can prevent detachment of the core wire 150 from the front end tip 30 when the core wire 150 moves in the long axis direction. The front end portion 53 may be formed with one (single) depressed portion into which a portion of the front end tip 30 enters.

As shown in Fig. 12, the front end portion 53 of the core wires 50, 150 may be tapered so as to become thinner toward the front end. This configuration can improve the flexibility of the front end portions 53 of the catheters 50, 150.

## Claims

1. A catheter (1, 101) having a long axis, the catheter (1, 101) comprising:
a hollow shaft (10) having a front end and a base end;
a mesh member (20) with a tubular shape, the mesh member having a base end joined to the front end of the hollow shaft (10), and being configured to expand and contract in a radial direction perpendicular to the long axis of the catheter (1, 101) ;
a front end tip (30) joined to a front end of the mesh member (20); and
a core wire (50, 150) having a long axis and a front end portion (53) joined to the front end tip (30), and extending through an inside of the mesh member (20) and the hollow shaft (10), **characterised in that**
the front end portion (53) of the core wire (50, 150) comprises one or more holes (54, 55, 56, 57, 58, 154, 155), and a portion of the front end tip (30) enters into at least a portion of the one or more holes (54, 55, 56, 57, 58, 154, 155) .

2. The catheter according to claim 1, wherein
the one or more holes are one or more through-holes (54, 55, 56, 57, 58), and
a portion of the front end tip (30) enters into at least a portion of the one or more through-holes (54, 55, 56, 57, 58) .

3. The catheter according to claim 2, wherein
the portion of the front end tip (30) penetrates through the one or more through-holes (54, 55, 56, 57, 58).

4. The catheter according to claim 2 or 3, wherein a width of the one through-hole (55) increases toward a front end of the core wire.

5. The catheter according to claim 1, wherein
the one or more holes are one or more depressed portions (154, 155), and
a portion of the front end tip (30) enters into at least a portion of the one or more depressed portions (154, 155).

6. The catheter according to claim 5, wherein each of the one or more depressed portions (155) is depressed in a direction intersecting the long axis of the core wire (150).

7. The catheter according to any one of claims 1 to 6, wherein a front end of the front end portion (53) is covered by the front end tip (30).

8. The catheter according to any one of claims 1 to 7, wherein the front end portion (53) has a plate-shape.

9. The catheter according to any one of claims 1 to 8, wherein the front end portion (53) of the core wire (50, 150) is tapered to become thinner toward a front end of the front end portion (53).

## Patentansprüche

1. Katheter (1, 101) mit einer Längsachse, der aufweist:
einen Hohlschaft (10), der ein vorderes Ende und ein hinteres Ende hat;
ein röhrenförmiges Maschenwerk (20), das ein an das vordere Ende des Hohlschafts (10) angefügtes hinteres Ende hat und so konfiguriert ist, dass es sich in Radialrichtung quer zur Längsachse des Katheters (1, 101) ausdehnt und zusammenzieht;
eine an ein vorderes Ende des Maschenwerks (20) angefügte Vorderendspitze (30); und
einen Kerndraht (50, 150), der eine Längsachse und einen an die Vorderendspitze (30) angefügten vorderen Endabschnitt (53) hat und sich durch das Maschenwerk (20) und den Hohlschaft (10) hindurch erstreckt, **dadurch gekennzeichnet, dass**
der vordere Endabschnitt (53) des Kerndrahts (50, 150) ein oder mehrere Löcher (54, 55, 56, 57, 58, 154, 155) aufweist, und ein Teil der Vorderendspitze (30) wenigstens teilweise in das eine oder die mehreren Löcher (54, 55, 56, 57, 58, 154, 155) eindringt.

2. Katheter nach Anspruch 1, wobei
das eine oder die mehreren Löcher ein oder mehrere Durchgangslöcher (54, 55, 56, 57, 58) sind, und
ein Teil der Vorderendspitze (30) wenigstens teilweise in das eine oder die mehreren Durchgangslöcher (54, 55, 56, 57, 58) eindringt.

3. Katheter nach Anspruch 2, wobei
der Teil der Vorderendspitze (30) das eine oder die mehreren Durchgangslöcher (54, 55, 56, 57, 58) durchdringt.

4. Katheter nach Anspruch 2 oder 3, wobei die Breite des einen Durchgangslochs (55) zum vorderen Ende des Kerndrahts hin zunimmt.

5. Katheter nach Anspruch 1, wobei
das eine oder die mehreren Löcher eine oder mehrere Vertiefungen (154, 155) sind, und
ein Teil der Vorderendspitze (30) wenigstens teileweise in die eine oder die mehreren Vertiefungen (154, 155) eindringt.

6. Katheter nach Anspruch 5, wobei
jede der einen oder mehreren Vertiefungen (155) in eine Richtung quer zur Längsachse des Kerndrahts (150) geht.

7. Katheter nach einem der Ansprüche 1 bis 6, wobei ein vorderes Ende des vorderen Endabschnitts (53) von der Vorderendspitze (30) verdeckt ist.

8. Katheter nach einem der Ansprüche 1 bis 7, wobei der vorderen Endabschnitt (53) plattenförmig ist.

9. Katheter nach einem der Ansprüche 1 bis 8, wobei der vordere Endabschnitt (53) des Kerndrahts (50, 150) sich in der Weise verjüngt, dass er zum vorderen Ende des vorderen Endabschnitts (53) hin dünner wird.

## Revendications

1. Cathéter (1, 101) ayant un axe long, le cathéter (1, 101) comprenant :
un arbre creux (10) ayant une extrémité avant et une extrémité de base ;
un élément maillé (20) de forme tubulaire, l'élément maillé ayant une extrémité de base jointe à l'extrémité avant de l'arbre creux (10), et étant configuré pour se dilater et se contracter dans une direction radiale perpendiculaire à l'axe long du cathéter (1, 101) ;
une pointe d'extrémité avant (30) jointe à une extrémité avant de l'élément maillé (20) ; et
un fil central (50, 150) ayant un axe long et une partie d'extrémité avant (53) jointe à la pointe d'extrémité avant (30), et s'étendant à travers un intérieur de l'élément maillé (20) et de l'arbre creux (10), **caractérisé en ce que**
la partie d'extrémité avant (53) du fil central (50, 150) comprend un ou plusieurs trous (54, 55, 56, 57, 58, 154, 155), et une partie de la pointe d'extrémité avant (30) s'engage dans au moins une partie dudit un ou plusieurs trous (54, 55, 56, 57, 58, 154, 155).

2. Cathéter selon la revendication 1, dans lequel
ledit un ou plusieurs trous sont un ou plusieurs trous traversants (54, 55, 56, 57, 58), et
une partie de la pointe d'extrémité avant (30) s'engage dans au moins une partie dudit un ou plusieurs trous traversants (54, 55, 56, 57, 58).

3. Cathéter selon la revendication 2, dans lequel
la partie de la pointe d'extrémité avant (30) pénètre à travers ledit un ou plusieurs trous traversants (54, 55, 56, 57, 58).

4. Cathéter selon la revendication 2 ou 3, dans lequel une largeur dudit un trou traversant (55) augmente vers une extrémité avant du fil central.

5. Cathéter selon la revendication 1, dans lequel
ledit un ou plusieurs trous sont une ou plusieurs parties enfoncées (154, 155), et
une partie de la pointe d'extrémité avant (30) s'engage dans au moins une partie de ladite une ou plusieurs parties enfoncées (154, 155).

6. Cathéter selon la revendication 5, dans lequel
chacune de ladite une ou plusieurs parties enfoncées (155) est enfoncée dans une direction coupant l'axe long du fil central (150).

7. Cathéter selon l'une quelconque des revendications 1 à 6, dans lequel une extrémité avant de la partie d'extrémité avant (53) est recouverte par la pointe d'extrémité avant (30).

8. Cathéter selon l'une quelconque des revendications 1 à 7, dans lequel la partie d'extrémité avant (53) a une forme de plaque.

9. Cathéter selon l'une quelconque des revendications 1 à 8, dans lequel la partie d'extrémité avant (53) du fil central (50, 150) est effilée pour devenir plus mince vers une extrémité avant de la partie d'extrémité avant (53).
